(19) Europäisches Patentamt
European Patent Office
Office européen des brevets

(11) Veröffentlichungsnummer: **0 449 170 A1**

(12) # EUROPÄISCHE PATENTANMELDUNG

(21) Anmeldenummer: **91104659.7**

(22) Anmeldetag: **25.03.91**

(51) Int. Cl.5: **C07K 13/00**, C12N 15/12, A61K 37/02

Der Anmelder hat nachträglich ein Sequenzprotokoll eingereicht und erklärt, dass dieses keine neuen Angaben enthält.

(30) Priorität: **30.03.90 DE 4010237**

(43) Veröffentlichungstag der Anmeldung: **02.10.91 Patentblatt 91/40**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**W-3550 Marburg 1(DE)**

(72) Erfinder: **Hoppe-Seyler, Felix, Dr.**
**Hirtenau 38**
**W-6900 Heidelberg(DE)**
Erfinder: **Hirt, Lorenz**
**Chemin de la Cure 15**
**CH-1012 Lausanne(CH)**
Erfinder: **Butz, Karin**
**Weinheimer Strasse 7**
**W-6945 Hirschberg(DE)**
Erfinder: **Bartsch, Dusan, Dr.**
**St. Annagasse 13**
**W-6900 Heidelberg(DE)**
Erfinder: **Bauknecht, Tobias**
**Ringstrasse 25**
**W-6900 Heidelberg(DE)**
Erfinder: **Royer, Hans D., Dr.**
**Friedlandstrasse 13**
**W-6915 Dossenheim(DE)**

(74) Vertreter: **Klein, Otto, Dr. et al**
**Hoechst AG Zentrale Patentabteilung**
**Postfach 80 03 20**
**W-6230 Frankfurt am Main 80(DE)**

(54) **Zelluläres Bindungsprotein mit Affinität für die regulatorische Region des menschlichen Papillomavirus Typ 18.**

(57) Die Erfindung betrifft eine DNA-Sequenz, die für das zelluläre DNA-bindende Protein RS1, das sich spezifisch an die 28 bp E2-Subregion der Upstream-Regulatory-Region (URR) des menschlichen Papillomavirus (HPV) bindet, kodiert sowie das Protein RS1 und RS1-ähnliche Proteine.

EP 0 449 170 A1

Die Erfindung betrifft DNA-Sequenzen, die für ein DNA-bindendes Protein (RS1) kodieren, sowie ein neues DNA-bindendes Protein (RS1), das mit Hilfe der rekombinanten DNA-Technik aus primären menschlichen Keratinozyten isoliert wurde.

Die Erfindung betrifft weiterhin rekombinante DNA-Moleküle zur Verwendung in der Herstellung von RS1-Protein oder RS1-ähnlichen Proteinen mit der biologischen Aktivität von RS1 sowie Wirtsorganismen wie Bakterien, Hefen und Säugerzellen, die mit diesen rekombinanten DNA-Molekülen transformiert sind.

Die Erfindung betrifft schließlich pharmazeutische Zusammensetzungen, die das RS1-Protein oder RS1-ähnliche Proteine mit der Aktivität von RS1 enthalten.

Papillomaviren sind kleine doppelsträngige DNA-Viren und sind verantwortlich für gutartige Haut- und Schleimhautepithellesionen. Außderdem wird aber auch eine Verbindung zwischen dem Auftreten von bestimmten Typen des menschlichen Papillomavirus (HPV) und anogenitalem Krebs (The Papovaviridae, Vol. 2, Plenum Publishing Corp., New York, Seiten 245-263) gesehen. Ein wichtiger Hinweis auf die Verbindung zwischen Krebsentstehung und HPV-Anwesenheit besteht darin, daß in über 90 % der Cervikalen Tumore nach Biopsie HPV-DNA-Sequenzen festgestellt wurden. Die meisten dieser Tumore enthalten HPV Typ 16 oder HPV Typ 18 DNA. HPV 18 DNA-Sequenzen wurden auch in einer Reihe von Zellen aus Zellinien, die aus cervikalen Tumoren gewonnen wurden, entdeckt. Sowohl in cervikalen Tumoren als auch in Zellinien, die von solchen Tumoren abstammen, ist die virale DNA gewöhnlich in das Wirtsgenom integriert. Es ist auch bekannt, daß in solchen Zellen die virale Transkription stattfindet. Die Untersuchung von etablierten Tumorzellinien legten die Vermutung nahe, daß es sich bei den HPV-Sequenzen um die verantwortlichen Sequenzen für die Entwicklung des Krebses handelt. Es gibt auch starke Hinweise darauf, daß die Expression der frühen offenen Leseraster (ORF) der E6 und E7 Gene der HPV-Typen 16 und 18, die für die Transformierungsaktivität (transforming activity) kodieren, eine entscheidende Rolle bei der Initiierung und bei der Aufrechterhaltung der transformierten Zellen spielen (Cancer Res. 48: 3780-3786).

Regulatorische Transkriptionselemente befinden sich in der sogenannten URR-Region (upstream - regulatory-region) des HPV-Genoms, die auch als die nicht kodierende Region (non-coding region) oder die lange Kontrollregion (long-control region) bezeichnet wurde. Diese Region erstreckt sich über 825 Basen-Paare vom Ende des späten offenen Leserasters (ORF L1) bis zum Anfang des frühaktiven Gens E6 in der HPV 18 DNA. Die HPV 18 URR-Region enthält einen Promoter, der in mehreren Tumorlinien aktiv ist und der wenigstens 3 verschiedene Verstärkerregionen (enhancer) besitzt (J. Virol. 62:665-672, 1988).

Die Aufgabe der vorliegenden Erfindung ergibt sich unter den folgenden Gesichtspunkten:
Die Regulation viraler Gene durch zelluläre Proteine hat eine Schlüsselrolle in der Tumorentwicklung. Die Deregulation der Expression der transformierten frühen Gene E6 und E7 vom HPV 16 und 18 trägt zum Prozeß der Krebsentstehung in den transformierten Zellen bei. Insofern ist die Identifikation, die Isolierung und Herstellung von Proteinen, die eine Bindungsaffinität für die regulatorische Region dieser frühen Gene besitzen, ein entscheidender Schritt in der Bekämpfung der obengenannten Tumore. Die die deregulatorischen Regionen bindenden Proteine stellen deshalb möglicherweise geeignete Mittel für die Herstellung eines Mittels zur Bekämpfung von Krebs dar.

Die vorliegende Erfindung hatte deshalb die Identifizierung von Faktoren, die die Regulation und Expression viraler DNA in infizierten menschlichen Zellen beeinflussen können und deren Bereitstellung in genügend großen Mengen, um sie als Mittel gegen Krebserkrankungen einsetzen zu können, zur Aufgabe.

Diese Aufgabe wurde erfindungsgemäß einerseits gelöst durch die Bereitstellung einer DNA-Sequenz, wie sie in der Figur 8 bzw. Anspruch 1 abgebildet ist, die für das zelluläre DNA-bindende Protein RS1, oder ein RS1-ähnliches Polypeptid mit der biologischen Aktivität von RS1, kodiert.

Andererseits wird zur Lösung der Aufgabe ein DNA-bindendes Protein (RS1) mit der in Figur 8 bzw. Anspruch 11 gezeigten Aminosäuresequenz und der Eigenschaft, spezifisch an die 28 bp E2-Region der Upstream-Regulatory-Region des HPV 18 zu binden, bereitgestellt.

Weiterhin umfaßt die Erfindung DNA-Sequenzen, die aus dem Genom von primären menschlichen Keratinozyten stammen und für das zelluläre DNA-bindende Protein RS1 kodiert.

Außerdem sind DNA-Sequenzen umfaßt, die mit einer der obengenannten DNA-Sequenz hybridisieren. Diese DNA-Sequenzen können natürlichen, halb-synthetischen oder synthetischen Ursprungs sein und stehen zu einer der oben genannten DNA-Sequenzen durch Mutation wie Nukleotid-Substitution, Nukleotid-Deletion, Nukleotid-Insertion oder Inversion von Nukleotid-Abschnitten in Beziehung. Weiterhin sind diese letztgenannten DNA-Sequenzen dadurch gekennzeichnet, daß sie für ein RS1-ähnliches Polypeptid mit der biologischen Aktivität von RS1 kodieren.

Eine biologische Aktivität von RS1 ist z. B. die spezielle Affinität (high affinity binding) von RS1 für die 28 bp lange E2-Subregion der upstream regulatory region der HPV-Typen 16 und 18. Als RS1-ähnliche Proteine werden Proteine bezeichnet, die wie das Protein RS1 eine Affinität für die DNA E2-Subregion der URR der HPV-Typen 16-18 besitzen.

Weiterhin umfaßt die Erfindung rekombinante DNA-Moleküle für die Klonierung in Vektoren, wobei die DNA-Sequenz aus der Gruppe der obengenannten DNA-Sequenzen ausgewählt ist, die für das zelluläre DNA-bindende Protein RS1 oder RS1-ähnliche Proteine kodieren.

Diese rekombinanten DNA-Moleküle werden mit einer Expressions-Kontroll-Sequenzoperativ verbunden.

Bevorzugt sind rekombinante DNA-Moleküle mit einer Expressions-Kontroll-Sequenz, ausgewählt aus einem E.coli-Promotersystem, wie dem E.coli-Lac-System, dem E.coli-ß-Lactamase-System, dem E.coli-trp-System, dem E.coli-Lipoproteinpromoter, oder mit einer Hefe-Expressions-Kontrollsequenz oder einer anderen eukaryotischen Expressions-Kontroll-Sequenz.

Weiterhin umfaßt die Erfindung Wirtsorganismen, die mit wenigstens einem dieser rekombinanten DNA-Moleküle transformiert werden.

Bevorzugt werden Wirtsorganismen verwendet, die aus folgender Gruppe ausgewählt sind: E.coli, ein anderes Bakterium, Hefe, ein anderer Pilz, eine tierische oder menschliche Zelle.

Besonders bevorzugt wird ein rekombinanter Lambda gt 11 benutzt. Das erfindungsgemäße DNA-bindende Protein RS1 hat die Eigenschaft, spezifisch an die 28 bp E2-Region der URR vom HPV-Typ 18 und 16 zu binden. Erfindungsgemäße DNA-bindende Proteine umfassen auch Proteine mit den biologischen Eigenschaften von RS1, also alle RS1-ähnlichen Proteine, die ebenfalls die Eigenschaft besitzen, an die E2-Region der URR vom HPV-Typ 18 und 16 zu binden, oder aber sie haben die Wirkung von RS1, nämlich durch spezifische Interaktion mit der E2-Region der URR oder überlappender Regionen wie die Regulation der frühen Gene E6 und E7 den HPV zu beeinflussen.

Die erfindungsgemäßen Proteine werden von einer der oben genannten erfindungsgemäßen DNA-Sequenzen kodiert und mit Hilfe der rekombinanten DNA-Technologie auf an sich bekannte Weise unter Verwendung eines erfindungsgemäßen Wirtsorganismus hergestellt.

Bei diesen Verfahren zur Herstellung von RS1 oder RS1-ähnlichen Polypeptiden mit der biologischen Aktivität von RS1 wird ein erfindungsgemäßer Wirtsorganismus mit einem erfindungsgemäßen rekombinanten DNA-Molekül transformiert. Der transformierte Organismus wird kultiviert und das RS1 oder RS1-ähnliche Polypeptid wird nach Expression aus dem Kulturmedium isoliert.

Nach diesen Verfahren erhältliche Proteine sind ebenfalls erfindungsgemäße Proteine.

Aufgrund der biologischen Aktivität der erfindungsgemäßen Proteine, die eine Beeinflussung der Regulation HPV-transformierter Zellen im Hinblick auf eine virale Expression zulassen, sind diese Proteine für die Krebstherapie geeignet.

Diese Erfindung umfaßt deshalb auch pharmazeutische Zusammensetzungen, die das Protein RS1 oder RS1-ähnliche Polypeptide enthalten. Diese Mittel können in der Krebs- oder Tumortherapie eingesetzt werden.

Bei dem Versuch der Identifizierung und Isolierung von URR-bindenden Proteinen wurde eine Strategie angewandt, die es erlaubt, die durch komplementäre DNA (cDNA's) kodierten Faktoren aufgrund ihrer sequenzspezifischen Bindungseigenschaft zu DNA-Fragmenten zu isolieren. Diese Methode beruht auf einer hohen Expressionsrate der den DNA-bindenden Faktor kodierenden, DNA-bindenden Region im Bakteriophagen lambda gt 11. Die Interaktion dieses Faktors mit doppelsträngiger radioaktiv markierter DNA führt zu einem Erkennungssignal. Danach kann die korrespondierende Rekombinante in der lambda gt 11 Expressions-Bibliothek isoliert werden.

Kurze Beschreibung der Figuren:

Fig. 1:

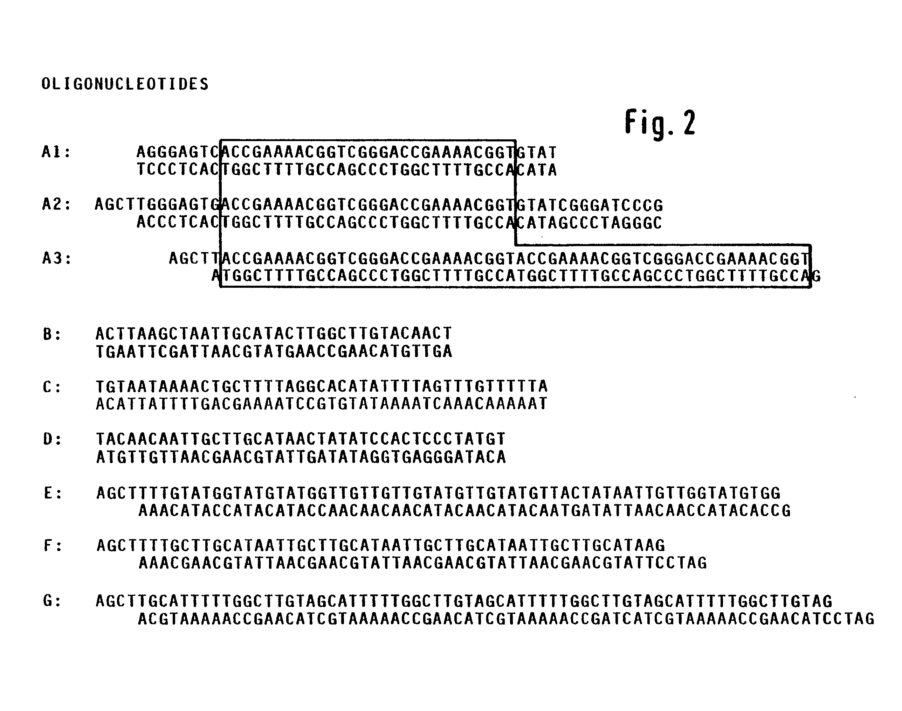

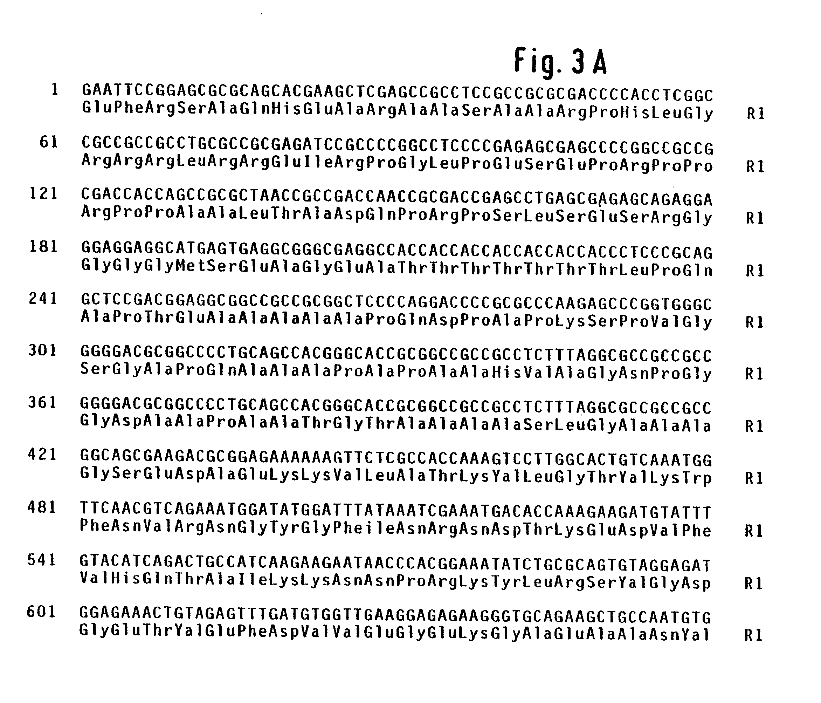

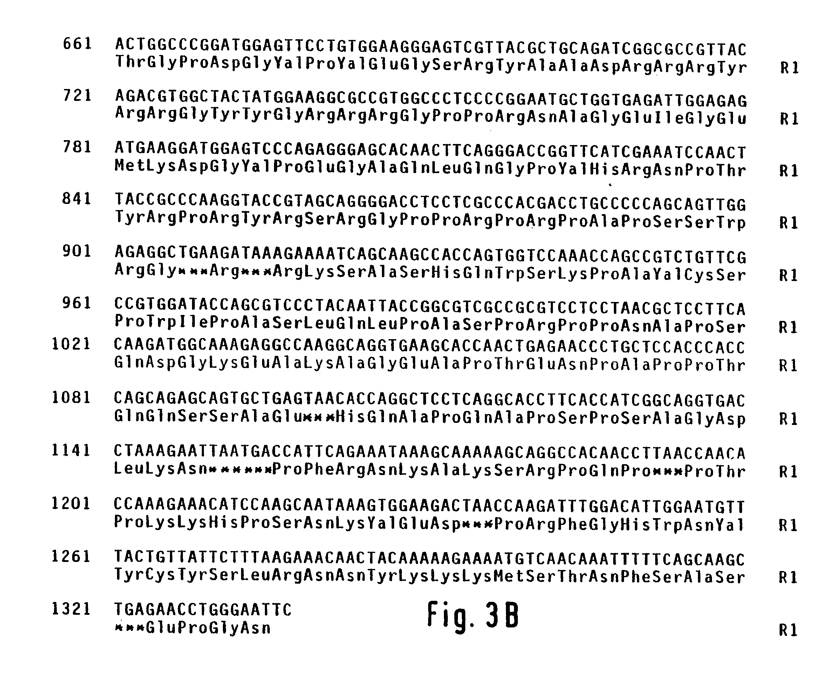

Schematisches Diagramm der regulatorischen Region URR (upstream-regulatory-region) mit menschlichem Papillomavirus HPV 18. CAAT-Box und TATA-Box sind durch eine Ellipse bzw. ein Rechteck markiert. Die E2-Subregion, die die zwei pallindromischen Wiederholungen (pallindromic repeats) enthält, ist darunter abgebildet. Die pallindromischen Wiederholungen sind durch Pfeile markiert. Sie stellen den Erkennungsbereich für die viralen E2-Proteine dar. Eingerahmt ist die Kernsequenz, die allen Nukleotiden (Fig. 2) eigen ist, die von dem DNA-Bindungsprotein RS1 gebunden wird.

Fig. 2:

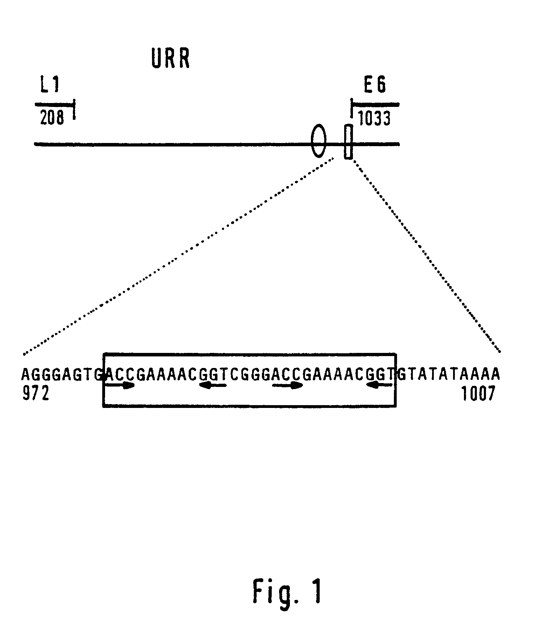

Oligonukleotide, die in dieser Erfindung benutzt wurden:
Oligonukleotide A1 bis A3 enthalten Sequenzen, die von der E2-Subregion, die in Figur 1 beschrieben ist, abstammen. Ihnen gemeinsam ist eine 28 Basen-Paare lange Kernsequenz (eingerahmt und verdoppelt in A3).

Oligonukleotide B bis F und G sind Monomere oder Tetramere, die von anderen Regionen der URR des HPV 18 bzw. HPV 16 stammen.

Fig. 3:

Sequenz der RS1 cDNA und des RS1-Proteins. Die Aminosäuresequenz ist in dem 3-Buchstabencode angegeben.

Isolation des cDNA-Klons cRS1:

Zur Isolation des zellulären DNA-bindenden Proteins RS1, das an die regulatorische Region URR des HPV 18 (Fig. 1) bindet, wurde eine lambda gt 11 Expressions-Bibliothek, die cDNA primärer Epidermalkeratinozyten enthält, die die natürlichen Wirtszellen des HPV's sind, getestet. Dabei wurde die Methode, die ursprünglich von Singh et al, Cell 52:415-423, 1988 appliziert wurde und von Vinson et al (Genes Dev. 1988 2:801-806) modifiziert wurde, angewandt. Als eine Sonde (probe) für die Auffindung von Bindungsproteinen wurde das Oligonukleotid A3 (Fig. 2) verwendet. Dieses Oligonukleotid enthält eine Duplikation von 2 Tandemwiederholungen (tandem-repeats), die die Erkennungsregion für den viralen E2-Transaktivator/Transrepressor (Nature 325:70-73, 1987) einschließen. In einem ersten Ansatz wurden 500.000 pfu der lambda gt 11 Expressions-Bibliothek getestet. PolydldC, das keine biologische Information enthält, wurde zur Verhinderung von unspezifischer Bindung verwendet. In einem weiteren Ansatz wurden weitere 500.000 pfu getestet, wobei bei diesem Ansatz denaturierte und beschallte Kalb-Thymus-DNA zur Verhinderung von unspezifischer Bindung verwendet wurde. Beide Testrunden (rounds of screening) führten lediglich zu einem positiven Signal, das zudem an derselben Stelle auf dem Original und dem Duplikatfilter auftrat. Eine Restriktionsanalyse und eine partielle Sequenzierung der cDNA-Fragmente (inserts) zeigte, daß beide Klone identisch sind und wahrscheinlich Duplikate des gleichen Phagens representieren. Der identifizierte Phage (bezeichnet als RS1-Phage) wurde durch 4 Plattierungsrunden gereinigt und danach detailliert analysiert.

Spezifität der Bindung:

Die Bindungsspezifität wurde analysiert mit Hilfe von Filterbindungstests, wobei 3 verschiedene Oligonukleotide (A1, A2 und A3) verwendet wurden, die alle die 28 Basen-Paare lange E2-Subregion enthalten. Im Vergleich dazu wurden mehrere Nukleotide, die nicht in Beziehung zur E2-Subregion stehen, aber von der regulatorischen Region URR des HPV 18 und HPV 16 stammen, als negative Kontrolle verwendet (Fig. 2). Konzentrierte Phagenproben (5 x $10^7$ pfu) wurden auf frischgegossenen E.coli Y 1090-Rasen gesprüht. Die DNA-Bindungseigenschaften von RS1 und von einem Produkt eines negativen Kontrollklons, der zufallsmäßig aus der lambda gt 11-Expressions-Bibliothek ausgewählt wurde, wurden mit Hilfe von Filterbindungstests analysiert. In allen Fällen interagierte das Produkt des negativen Kontrollklons nicht mit einem der getesteten Oligonukleotide. Im Gegensatz dazu band RS1 spezifisch an die Oligonukleotide A1, A2 und A3. Eine Interaktion zwischen RS1 und anderen Kontrolloligonukleotiden wurde nicht beobachtet.

Es stellte sich auch heraus, daß RS1 eine hohe und spezifische Bindungsaktivität zu einzelsträngiger DNA, und zwar zum nicht codogenen (anti-sense) Strang (Fig. 2) der Oligonukleotide A1, A2 und A3 besitzt. Eine Affinität von RS1 zu dem codogenen (sense)-Strang der Oligonukleotide A1, A2 und A3 oder zu einem anderen Einzelstrang der Kontrolloligonukleotide wurde nicht beobachtet. Der negative Kontrollphage zeigte keinen Faktor, der eine Aktivität zu einzelsträngiger DNA besitzt.

RS1 bindet auch bei hohen Salzkonzentrationen (400 mM NaCl) an DNA, was eine hohe Bindungsspezifität zeigt.

Auch unter hohen Salzkonzentrationen war eine Bindung von RS1 ausschließlich an Oligonukleotide zu beobachten, die die 28 Basen-Paare lange Kernsequenz enthalten. Keine Interaktion mit anderen doppelsträngigen oder einzelsträngigen DNA-Fragmenten wurde beobachtet.

Analyse des von dem RS1-Phagen kodierten ß-Galactosidasefusionsproteins:

Lysogene RS1-Phagen und negative Kontrollphagen wurden isoliert und zur Produktion von großen Mengen ihres entsprechenden ß-Galactosidasefusionsproteins induziert. Western-Blots-Proteine von induzierten und nicht-induzierten lysogenen Phagen wurden mit anti-ß-Galactosidase-Maus-Antikörpern behandelt und danach mit einem Anti-Maus-Antikörper, der kovalent an alkalischer Phosphatase gebunden wurde, beschichtet. IPTG induzierte ein Fusionsprotein von ungefähr 150 K in dem lysogenen RS1-Phagen.

Die Bindungseigenschaften des ß-Gal-RS1-Fusionsproteins wurden mit Hilfe von South-Western-Analyse des gesamten Proteinextrakts von induzierten und nicht induzierten lysogenen Kulturen überprüft. Nach dem Transfer wurden die immobilisierten Proteine 60 min lang mit 7 M Guanidinhydrochlorid denaturiert und danach 24 h lang renaturiert. Science (1988) 241:577-580. Der Denaturierungs/Renaturierungs-Zyklus verbesserte die Erkennungssignale der Proteine bei der South-Western-Analyse und das Gesamtprotein von induzierten lysogenen Phagen einer negativen Kontrolle und RS1 wurde mit Hilfe der radioaktiv markierten Sonde Oligonukleotid A3b analysiert. Eine einzelne Bande, die ein Protein mit einer molekularen Masse von ungefähr 150 K repräsentiert, wurde spezifisch im lysogenen Phagen RS1 nachgewiesen. Die Spezifität der DNA-Bindung durch dieses 150 K-Fusionsprotein der South-Western-Analyse sicherte zusätzlich die Bindungsspezifität von RS1, wie sie in den Filterbindungstests nachgewiesen wurde, ab. Das 150 K-Protein bindet spezifisch an die Oligonokleotide A1, A2 und A3 (Fig. 2) und an ihre entsprechenden nicht-codogenen (anti-sense ) Stränge und bindet nicht an die Kontrolloligonukleotide.

Struktur der im Phagen RS1 vorliegenden cDNA:

Der Phage RS1 enthält ein cDNA-Fragment mit einer Länge von 1338 Nukleotiden. Der eine Strang beginnt an seinem 5'-Ende mit einem offenen Leseraster (ORF) von 906 Nukleotiden, gefolgt von einer 3'-nicht translatierten Region von 432 Nukleotiden. Das 906 Nukleotide lange ORF, das 302 Aminosäuren entspricht, ist am 3'-Ende im Raster (in frame) an das ß-Galactosidase-Gen gebunden. Das ß-Galactosidase-RS1-Fusionsprotein hat eine molekulare Masse von ungefähr 150 K. Der ß-Galactosidase-Anteil an diesem Fusionsprotein hat eine molekulare Masse von ungefähr 120 kD, so daß dadurch der cDNA-kodierte Anteil am Fusionsprotein eine molekulare Masse von ungefähr 30 kD besitzen muß.

Diese Ergebnisse stimmen überein mit dem Molekulargewicht von 33,6 H, das für ein Oligopeptid mit 302 Amionsäuren berechnet werden kann, die von dem 906 Nukleotide langen ORF der RS1-Phagen cDNA kodiert werden. In dem 906 Nukleotide langen ORF ist ein potentielles Translations-Initiierungscodon an der Base 192 vorhanden. Jedoch gehorchen die umgebenden Basen nicht der Kozak-Konsensus-Sequenz für eukaryotische Translations-Initiierungsstellen (Cell 44: 283-292). Deshalb mag es möglich sein, daß das Codon AUG in Position 192 nicht das ursprüngliche Translations-Initiationskodon für das RS1-Gen darstellt. Zwei Polyadenylationskonsensus-Sequenzen (AATAAA) sind in der 3'-Region der RS1 cDNA vorhanden und beginnen an den Nukleotiden 1167 bzw. 1221. Jedoch folgt ihnen kein Poly(A)-Schwanz.

Ein Vergleich der RS1 cDNA-Sequenz bzw. der von dieser deduzierten Proteinsequenz mit mehreren Sequenz-Datenbanken (Gene bank, Swiss Prot, Dayhoff) zeigte keine signifikante Nukleotid- oder Aminosäuresequenzhomologie von RS1 mit bekannten Proteinen. Die Aminosäuresequenz von RS1 zeigt mehrere interessante Merkmale. Vor allem ist das Protein reich an Arginin (34 von 302 Aminosäuren) und an Prolin (35 von 302 Aminosäuren). Prolin kommt teilweise in Form von Nestern vor (z.B. Aminosäuren 278-296). Diese Prolinnester sind bekanntlich an der Aktivierung der Transkription durch mehrere Transkriptionsfaktoren beteiligt. Eine Region von 51 Aminosäuren (33-84) ist reich an Prolin (20 %), Glutaminsäure (10 %), Serin (10 %) und Threonin (18 %). Analoge Regionen sind in der Aminosäuresequenz einer Gruppe von Proteinen mit einer sehr kurzen Halbwertzeit in vivo ("PEST"-proteins) vorhanden. Die Reste 10 bis 51 haben einen hohen Gehalt an der basischen Aminosäure Arginin (20 %). RS1 enthält auch keine Region, die Zinkfinger, Homeoboxen oder Leucin-Zipper, die Domänen darstellen, wie sie in mehreren anderen DNA-bindenden Faktoren vorhanden sind, enthalten (Science 1989, 245: 371-378).

Beispiel 1

Eine cDNA-Expressions-Bibliothek

Eine cDNA-Bibliothek von menschlichen epidermalen Keratinozyten, die in den Expressions-Vektor lambda gt 11 geklont ist, wurde von der Firma Clontech Laboratories, Inc. erhalten. Die Bibliothek enthielt ungefähr 1,7 x $10^6$ unabhängige rekombinante Klone mit einer durchschnittlichen Fragment (insert)-Größe von 1,1 kb.

Das Screening der lambda gt 11-Rekombinanten wurde mit Hilfe des Wirtsorganismus E.coli Y 1090 durchgeführt. Lysogene Phagen wurden in E.coli Y 1089 oder Y 1090 erzeugt (in: DNA-Cloning - A Practical Approach, Vol. 1, D.M. Glover, ed. (Oxford: IRL Press), 49-78).

Beispiel 2

DNA-Sonden und Test auf DNA-bindende Proteine

Oligonukleotide wurden mit einem Applied Biosystems 380A-Synthesizer mit Hilfe der Standard-Phosphorimidittechnik hergestellt. Die Produkte wurden mittels Gelelektrophorese gereinigt. Danach wurden sie unter Standardbedingungen mit ($^{32}P$) dATP mit Hilfe von Polynukleotidkinase (Firma Boehringer Mannheim) markiert. Beschallte Kalbthymus-DNA (Firma Pharmacia) und Poly dIdC (Firma Pharmacia) wurden zur Vermeidung von unspezifischer Bindung verwendet.

Der Test auf DNA-bindende Proteine erfolgte auf folgende Weise:

Lambda gt 11-Rekombinanten wurden in 0,7 % Agarose mit einem maximalen pfu von 1,5 x $10^5$/600 $cm^3$ in einem Nunc Bioassay Dish ausplattiert. Isopropyl-ß-D-thioglucopyranosid (10 $\mu$M)-gesättigte Nitrozellulosefilter (PA 85, Schleicher und Schuell) wurden 3 h lang bei 42 °C überschichtet. Nach einer weiteren 6-stündigen Inkubation bei 37 °C wurden die Filter entfernt und ein weiterer Filter wurde 3 h lang bei 37 °C darübergelegt. Die Filter wurden 10 min lang bei Raumtemperatur getrocknet und dann in Puffer A (50 mM NaCl, 0,5 mM Dithiotreitol (DTT), 25 mM Hepes (pH 7,9) und 6 M Guanidinhydrochlorid) inkubiert. Nach 10 min leichtem Schütteln wurde die Lösung durch den gleichen Puffer ersetzt. Nach weiteren 10 min wurde die Lösung schrittweise (Genes Dev.: 1988 2:801-806) viermal 5 min lang mit demselben Volumen Puffer B (50 mM NaCl, 0,5 mM DDT und 25 mM Hepes (pH 7,9) verdünnt.

Schließlich wurden die Filter zweimal 5 min lang mit Puffer B gewaschen und danach 30 min lang in Puffer C (5 % C arnation nonfat dry milk, 50 mM NaCl, 0,5 mM DTT und 25 mM Hepes (pH 7,9)) bei 4 °C inkubiert. Die Lösung wurde durch Puffer D (0,25 % Carnation nonfat dry milk, 50 mM NaCl, 0,5 mM DTT und 25 mM Hepes (pH 7,9)) ersetzt und 1 min lang inkubiert. Die eigentliche Bindungsreaktion wurde in Puffer D ausgeführt, der zusätzlich 3 x $10^6$ cpm/ml (1 x $10^8$ cpm/$\mu$g) markiertes doppelsträngiges Oligonukleotid und 10 $\mu$g/ml unspezifische DNA (denaturierte beschallte Kalbthymus-DNA, durchschnittliche Länge: 3000 bp oder PolydIdC, durchschnittliche Länge: 8260 bp) enthielt. Nach 60 min bei 4 °C wurden die Filter dreimal 5 min lang mit Puffer D gewaschen, mit Hilfe von 3 MM-Papier getrocknet und dann einem Kodak x-omat AR-Film nachts bei -70 °C unter Zuhilfenahme einer intensivierenden Blende ausgesetzt.

Beispiel 3:

Analyse des ß-Galactosidasefusionsproteins

Lysogene Phagen wurden nach der Methode von Huynh (DNA Cloning - A Practical Approach, Vol. 1, Oxford: IRL Press, 49-78) hergestellt. Die Proteinextrakte von lysogenen Phagen wurden präpariert (Cell 1988 52:415-423) und bei 70 °C in Aliquoten gelagert. Eine Western-Blot-Analyse wurde mit Hilfe einer Anti-ß-Galactosidase-Maus monoklonalen Antikörpers (Firma Promega) und einem "phosphatase based ProtoBlot Western Blot AP system (Promega)" unter den vom Anbieter empfohlenen Bedingungen ausgeführt. Zur Durchführung von South-Western-Analysen wurden die Proteinextrakte (50 $\mu$g Rohextrakt) 5 min im sample buffer gekocht (Proc. Natl. Acad. Sci. USA 82: 6741-6744) und danach auf ein 10 %-iges SDS PAGE-Gel geladen. Nach der Elektrophorese wurden die Proteine auf Nitrozellulose mit Hilfe von Electroblotting (semi-dry-system model SD1, CTI GmbH) übertragen. Die Filter wurden dann behandelt, wie es in Science 1988 241:577-580 beschrieben ist.

Beispiel 4:

Sequenzierung:

Die Fragmente (inserts) der rekombinanten Phagen wurden mit Hilfe des lambda-Phagen-Adsorptionssystems (Promega) hergestellt. Die inserts wurden in M13mp18 subkloniert und danach sequenziert mit Hilfe der Dideoxymethode (SEQUENASE; United States Biochemical, Inc.). Beide Stränge wurden vollständig sequenziert.

Beispiel 5:

Northern und Southern Blot Hybridisierung:

DNA wurde von HeLa, CGI3 (tumorigenic HeLa-fibroblast hybrids, (Science 1982 215:252-259)), 444 (nontumorigenic HeLa-fibroblast hybrids), primäre menschliche Fibroblasten, SV 80 (SV 40-transformed fibroblasts), HaCat (spontaneously immortalized keratinocytes (Journal of Cell Biology, 1988, 106:761-771)), Caski (cervical carcinoma), SW 480 (colon carcinoma), Wilms, Hep G2 (hepatoma) und menschlichen TK (osteosarcoma) Zellinien isoliert und zwar nach der Methode, die in Molecular Cloning: A Laboratory Manual: Cold Spring Harbor Laboratory beschrieben ist. Restriction digests wurden auf einem 1 %-igen Agarosegel aufgetrennt und dann auf GeneScreen Plus membranes (Du Pont) transferiert. Die Filter wurden vorhybridisiert und hybridisiert mit der $^{32}P$-markierten RS1 cDNA unter stringenten Bedingungen.

Zytoplasmatische RNA wurde nach der Methode, die in Cancer Research 1988, 48: 3780-3786 beschrieben ist, isoliert. Northern Blot Analyse wurde mit ungefähr 10 µg zytoplasmatischer RNA, die auf einem 1 %-igen Agarosegel aufgetrennt war, durchgeführt. Der Gellaufpuffer bestand aus 0,2 M Morpholinopropansulfonsäure pH 7,0; 50 mM Natriumacetat und 1 mM EDTA pH 8,0. Die aufgetrennte zytoplasmatische RNA wurde dann auf GeneScreen Plus Filter (Du Pont) transferiert. Die Hybridisierung und das Waschen der Filter und stringenten Bedingungen mit zufällig geprimten $^{32-P}$-markierten Sonden erfolgte in an sich bekannter Weise.

Beispiel 6:

Filterbindungstest:

Konzentrierte (1 x $10^{10}$ pfu/ml) Phagen-Lösung von Plaque-Lysaten wurde direkt auf frisch gegossenen E.coli Y 1090 Wirtszellrasen gesprüht. Nach einer 2-stündigen Inkubation bei 42 °C wurde diese Kultur mit Nitrozellulosefiltern, die IPTG (10 µM) gesättigt waren, beschichtet. Die Inkubation bei 37 °C wurde dann 2 h lang fortgesetzt. Danach wurden die Filter gesammelt und behandelt wie es in Beispiel 2 beschrieben ist.

## Patentansprüche

1. DNA-Sequenz

1 GAATTCCGGAGCGCGCAGCACGAAGCTCGAGCCGCCTCCGCCGCGCGACCCCACCTCGGC

61 CGCCGCCGCCTGCGCCGCGAGATCCGCCCCGGCCTCCCCGAGAGCGAGCCCCGGCCGCCG

121 CGACCACCAGCCGCGCTAACCGCCGACCAACCGCGACCGAGCCTGAGCGAGAGCAGAGGA

181 GGAGGAGGCATGAGTGAGGCGGGCGAGGCCACCACCACCACCACCACCACCCTCCCGCAG

241 GCTCCGACGGAGGCGGCCGCCGCGGCTCCCCAGGACCCCGCGCCCAAGAGCCCGGTGGGC

301 AGCGGTGCGCCCCAGGCCGCGGCCCCGGCGCCCGCCGCCCACGTCGCAGGAAACCCCGGT

361 GGGGACGCGGCCCCTGCAGCCACGGGCACCGCGGCCGCCGCCTCTTTAGGCGCCGCCGCC

421 GGCAGCGAAGACGCGGAGAAAAAAGTTCTCGCCACCAAAGTCCTTGGCACTGTCAAATGG

481 TTCAACGTCAGAAATGGATATGGATTTATAAATCGAAATGACACCAAAGAAGATGTATTT

541 GTACATCAGACTGCCATCAAGAAGAATAACCCACGGAAATATCTGCGCAGTGTAGGAGAT

601 GGAGAAACTGTAGAGTTTGATGTGGTTGAAGGAGAGAAGGGTGCAGAAGCTGCCAATGTG

661 ACTGGCCCGGATGGAGTTCCTGTGGAAGGGAGTCGTTACGCTGCAGATCGGCGCCGTTAC

721 AGACGTGGCTACTATGGAAGGCGCCGTGGCCCTCCCCGGAATGCTGGTGAGATTGGAGAG

781 ATGAAGGATGGAGTCCCAGAGGGAGCACAACTTCAGGGACCGGTTCATCGAAATCCAACT

841 TACCGCCCAAGGTACCGTAGCAGGGGACCTCCTCGCCCACGACCTGCCCCCAGCAGTTGG

901 AGAGGCTGAAGATAAAGAAAATCAGCAAGCCACCAGTGGTCCAAACCAGCCGTCTGTTCG

961 CCGTGGATACCAGCGTCCCTACAATTACCGGCGTCGCCGCGTCCTCCTAACGCTCCTTCA

1021 CAAGATGGCAAAGAGGCCAAGGCAGGTGAAGCACCAACTGAGAACCCTGCTCCACCCACC

1081 CAGCAGAGCAGTGCTGAGTAACACCAGGCTCCTCAGGCACCTTCACCATCGGCAGGTGAC

1141 CTAAAGAATTAATGACCATTCAGAAATAAAGCAAAAAGCAGGCCACAACCTTAACCAACA

1201 CCAAAGAAACATCCAAGCAATAAAGTGGAAGACTAACCAAGATTTGGACATTGGAATGTT

1261 TACTGTTATTCTTTAAGAAACAACTACAAAAAGAAAATGTCAACAAATTTTTCAGCAAGC

1321 TGAGAACCTGGGAATTC

die für das zelluläre DNA-bindende Protein RS1, oder ein RS1-ähnliches Polypeptid mit der biologischen Aktivität von RS1 kodiert.

2. DNA-Sequenz, dadurch gekennzeichnet, daß sie aus dem Genom von primären menschlichen Keratinozyten stammt und für das zelluläre Bindungs-Protein RS1 oder ein RS1-ähnliches Polypeptid mit der biologischen Aktivität von RS1 kodiert.

3. DNA-Sequenz, dadurch gekennzeichnet, daß sie mit einer DNA-Sequenz nach den Ansprüchen 1 und 2 hybridisiert, daß sie natürlichen, halb-synthetischen oder synthetischen Ursprungs ist, daß sie in Beziehung zu einer DNA-Sequenz nach den Ansprüchen 1 oder 2 steht durch Mutation wie Nukleotid-Substitution, Nukleotid-Deletion, Nukleotid-Insertion oder Inversion von Nukleotid-Abschnitten, und daß sie für ein RS1-ähnliches Polypeptid mit der biologischen Aktivität von RS1 kodiert.

4. Rekombinantes DNA-Molekül für die Klonierung, dadurch gekennzeichnet, daß die DNA-Sequenz nach einem der Ansprüche 1 bis 3 ausgewählt ist.

5. Rekombinantes DNA-Molekül nach Anspruch 4, dadurch gekennzeichnet, daß die DNA-Sequenz mit einer Expressions-Kontroll-Sequenz operativ verbunden ist.

6. Rekombinantes DNA-Molekül nach Anspruch 5, dadurch gekennzeichnet, daß die Expresssions-Kontroll-Sequenz ausgewählt ist aus einem E.coli-Promotersystem, dem E.coli-Lac-System, dem E.coli-ß-Lactamase-System, dem E.coli-trp-System, dem E.coli-Lipoproteinpromoter, einer Hefe-Expressions-Kontrollsequenz oder einer anderen eukaryotischen Expressions-Kontroll-Sequenz.

7. Wirtsorganismus, dadurch gekennzeichnet, daß er eine DNA-Sequenz, die für das DNA-bindende Protein RS1 kodiert, enthält.

8. Wirtsorganismus nach Anspruch 7, dadurch gekennzeichnet, daß er mit wenigstens einem der rekombinanten DNA-Moleküle nach einem der Ansprüche 4 bis 6 transformiert ist.

9. Wirtsorganismus nach Anspruch 8, dadurch gekennzeichnet, daß er ausgewählt ist aus der Gruppe E.coli, einem anderen Bakterium, Hefe, einem anderen Pilz, einer tierischen oder menschlichen Zelle.

10. Wirtsorganismus nach Anspruch 9, dadurch gekennzeichnet, daß es sich um Lambda gt 11 handelt.

11. DNA-bindendes Protein RS1 mit der folgenden Aminosäuresequenz.

GluPheArgSerAlaGlnHisGluAlaArgAlaAlaSerAlaAlaArgProHisLeuGly R1

ArgArgArgLeuArgArgGluIleArgProGlyLeuProGluSerGluProArgProPro R1

ArgProProAlaAlaLeuThrAlaAspGlnProArgProSerLeuSerGluSerArgGly R1

GlyGlyGlyMetSerGluAlaGlyGluAlaThrThrThrThrThrThrThrLeuProGln R1

AlaProThrGluAlaAlaAlaAlaAlaProGlnAspProAlaProLysSerProValGly R1

SerGlyAlaProGlnAlaAlaAlaProAlaProAlaAlaHisValAlaGlyAsnProGly R1

GlyAspAlaAlaProAlaAlaThrGlyThrAlaAlaAlaAlaSerLeuGlyAlaAlaAla R1

GlySerGluAspAlaGluLysLysValLeuAlaThrLysValLeuGlyThrValLysTrp R1

PheAsnValArgAsnGlyTyrGlyPheIleAsnArgAsnAspThrLysGluAspValPhe R1

ValHisGlnThrAlaIleLysLysAsnAsnProArgLysTyrLeuArgSerValGlyAsp R1

GlyGluThrValGluPheAspValValGluGlyGluLysGlyAlaGluAlaAlaAsnVal R1

ThrGlyProAspGlyValProValGluGlySerArgTyrAlaAlaAspArgArgArgTyr R1

ArgArgGlyTyrTyrGlyArgArgArgGlyProProArgAsnAlaGlyGluIleGlyGlu R1

MetLysAspGlyValProGluGlyAlaGlnLeuGlnGlyProValHisArgAsnProThr R1

TyrArgProArgTyrArgSerArgGlyProProArgProArgProAlaProSerSerTrp R1

ArgGly***Arg***ArgLysSerAlaSerHisGlnTrpSerLysProAlaValCysSer R1

ProTrpIleProAlaSerLeuGlnLeuProAlaSerProArgProProAsnAlaProSer R1

GlnAspGlyLysGluAlaLysAlaGlyGluAlaProThrGluAsnProAlaProProThr R1

GlnGlnSerSerAlaGlu***HisGlnAlaProGlnAlaProSerProSerAlaGlyAsp R1

LeuLysAsn******ProPheArgAsnLysAlaLysSerArgProGlnPro***ProThr R1

ProLysLysHisProSerAsnLysValGluAsp***ProArgPheGlyHisTrpAsnVal R1

TyrCysTyrSerLeuArgAsnAsnTyrLysLysLysMetSerThrAsnPheSerAlaSer R1

***GluProGlyAsn R1

12. DNA-bindendes Protein mit der Eigenschaft, spezifisch an die 28 bp E2-Subregion der Upstream-Regulatory-Region des menschlichen Papillomavirus HPV 18, die die Basensequenz ACCGAAAACGGTCGGGACCGAAAACGGT besitzt, zu binden.

13. DNA-bindendes Protein mit der biologischen Aktivität von RS1, dadurch gekennzeichnet, daß es von einer DNA-Sequenz nach einem der Ansprüche 1 bis 3 kodiert wird.

14. DNA-bindendes Protein, dadurch gekennzeichnet, daß es mit Hilfe der rekombinanten DNA-Technologie unter Verwendung eines Wirtsorganismus der Ansprüche 7 bis 10 hergestellt wird.

15. Verfahren zur Herstellung von RS1 oder RS1-ähnlichen Polypeptiden mit der biologischen Aktivität von RS1, dadurch gekennzeichnet, daß ein Wirtsorganismus mit einem rekombinanten DNA-Molekül nach den Ansprüchen 5 oder 6 transformiert wird, der transformierte Organismus kultiviert wird und das RS1 oder RS1-ähnliche Polypeptid nach Expression isoliert wird.

16. Polypeptid mit der biologischen Aktivität von RS1, dadurch gekennzeichnet, daß es nach einem Verfahren nach Anspruch 15 erhältlich ist.

17. Pharmazeutische Zusammensetzung, enthaltend das Polypeptid RS1 oder RS1-ähnliche Polypeptide mit der biologischen Aktivität von RS1 nach den Ansprüchen 11 bis 14 oder 16 als Mittel in der Krebs- oder Tumortherapie.

**Patentansprüche für folgenden Vertragsstaat: ES**

1. Verfahren zur Herstellung von RS1 oder RS1-ähnlichen Polypeptiden mit der biologischen Aktivität von RS1, dadurch gekennzeichnet, daß ein Wirtsorganismus mit einem rekombinanten DNA Molekül, das
   (1) eine DNA-Sequenz

```
  1 GAATTCCGGAGCGCGCAGCACGAAGCTCGAGCCGCCTCCGCCGCGCGACCCCACCTCGGC

 61 CGCCGCCGCCTGCGCCGCGAGATCCGCCCCGGCCTCCCCGAGAGCGAGCCCCGGCCGCCG

121 CGACCACCAGCCGCGCTAACCGCCGACCAACCGCGACCGAGCCTGAGCGAGAGCAGAGGA

181 GGAGGAGGCATGAGTGAGGCGGGCGAGGCCACCACCACCACCACCACCACCCTCCCGCAG

241 GCTCCGACGGAGGCGGCCGCCGCGGCTCCCCAGGACCCCGCGCCCAAGAGCCCGGTGGGC

301 AGCGGTGCGCCCCCAGGCCGCGGCCCCGGCGCCCGCCGCCCACGTCGCAGGAAACCCCGGT

361 GGGGACGCGGCCCCCTGCAGCCACGGGCACCGCGGCCGCCGCCTCTTTAGGCGCCGCCGCC

421 GGCAGCGAAGACGCGGAGAAAAAAGTTCTCGCCACCAAAGTCCTTGGCACTGTCAAATGG

481 TTCAACGTCAGAAATGGATATGGATTTATAAATCGAAATGACACCAAAGAAGATGTATTT

541 GTACATCAGACTGCCATCAAGAAGAATAACCCACGGAAATATCTGCGCAGTGTAGGAGAT

601 GGAGAAACTGTAGAGTTTGATGTGGTTGAAGGAGAGAAGGGTGCAGAAGCTGCCAATGTG

661 ACTGGCCCGGATGGAGTTCCTGTGGAAGGGAGTCGTTACGCTGCAGATCGGCGCCGTTAC

721 AGACGTGGCTACTATGGAAGGCGCCGTGGCCCTCCCCGGAATGCTGGTGAGATTGGAGAG
```

781 ATGAAGGATGGAGTCCCAGAGGGAGCACAACTTCAGGGACCGGTTCATCGAAATCCAACT

841 TACCGCCCAAGGTACCGTAGCAGGGGACCTCCTCGCCCACGACCTGCCCCCAGCAGTTGG

901 AGAGGCTGAAGATAAAGAAAATCAGCAAGCCACCAGTGGTCCAAACCAGCCGTCTGTTCG

961 CCGTGGATACCAGCGTCCCTACAATTACCGGCGTCGCCGCGTCCTCCTAACGCTCCTTCA

1021 CAAGATGGCAAAGAGGCCAAGGCAGGTGAAGCACCAACTGAGAACCCTGCTCCACCCACC

1081 CAGCAGAGCAGTGCTGAGTAACACCAGGCTCCTCAGGCACCTTCACCATCGGCAGGTGAC

1141 CTAAAGAATTAATGACCATTCAGAAATAAAGCAAAAAGCAGGCCACAACCTTAACCAACA

1201 CCAAAGAAACATCCAAGCAATAAAGTGGAAGACTAACCAAGATTTGGACATTGGAATGTT

1261 TACTGTTATTCTTTAAGAAACAACTACAAAAAGAAAATGTCAACAAATTTTTCAGCAAGC

1321 TGAGAACCTGGGAATTC

oder

(2) eine DNA-Sequenz aus dem Genom von primären mensohlichen Keratinozyten kodierend für das zelluläre Bindungsprotein RS1 oder ein RS1-ähnliches Polypeptid mit der biologischen Aktivität von RS1

enthält,

transformiert wird, der transformierte Organismus kultiviert wird und das RS1 oder RS1-ähnliche Polypeptid nach Expression isoliert wird.

2. Verfahren zur Herstellung einer pharmazeutischen Zusammensetzung, dadurch gekennzeichnet, daß das nach Anspruch 1 hergestellte RS1 oder RS1-ähnliche Polypeptid mit geeigneten Trägermaterialien gemischt wird.

**Patentansprüche für folgenden Vertragsstaat: GR**

1. DNA-Sequenz

1 GAATTCCGGAGCGCGCAGCACGAAGCTCGAGCCGCCTCCGCCGCGCGACCCCACCTCGGC

61 CGCCGCCGCCTGCGCCGCGAGATCCGCCCCGGCCTCCCCGAGAGCGAGCCCCGGCCGCCG

121 CGACCACCAGCCGCGCTAACCGCCGACCAACCGCCGACCGAGCCTGAGCGAGAGCAGAGGA

181 GGAGGAGGCATGAGTGAGGCGGGCGAGGCCACCACCACCACCACCACCACCCTCCCGCAG

241 GCTCCGACGGAGGCGGCCGCCCGCGGCTCCCAGGACCCCGCGCCCAAGAGCCCGGTGGGC

301 AGCGGTGCGCCCCAGGCCGCGGCCCCGGCGCCCGCCGCCCACGTCGCAGGAAACCCCGGT

361 GGGGACGCGGCCCCTGCAGCCACGGGCACCGCGGCCGCCGCCTCTTTAGGCGCCGCCGCC

421 GGCAGCGAAGACGCGGAGAAAAAAGTTCTCGCCACCAAAGTCCTTGGCACTGTCAAATGG

481 TTCAACGTCAGAAATGGATATGGATTTATAAATCGAAATGACACCAAAGAAGATGTATTT

541 GTACATCAGACTGCCATCAAGAAGAATAACCCACGGAAATATCTGCGCAGTGTAGGAGAT

601 GGAGAAACTGTAGAGTTTGATGTGGTTGAAGGAGAGAAGGGTGCAGAAGCTGCCAATGTG

661 ACTGGCCCGGATGGAGTTCCTGTGGAAGGGAGTCGTTACGCTGCAGATCGGCGCCCGTTAC

721 AGACGTGGCTACTATGGAAGGCGCCGTGGCCCTCCCCGAATGCTGGTGAGATTGGAGAG

781 ATGAAGGATGGAGTCCCAGAGGGAGCACAACTTCAGGGACCGGTTCATCGAAATCCAACT

841 TACCGCCCAAGGTACCGTAGCAGGGGACCTCCTCGCCCACGACCTGCCCCCAGCAGTTGG

901 AGAGGCTGAAGATAAAGAAAATCAGCAAGCCACCAGTGGTCCAAACCAGCCGTCTGTTCG

961 CCGTGGATACCAGCGTCCCTACAATTACCGGCGTCGCCCCGTCCTCCTAACGCTCCTTCA

1021 CAAGATGGCAAAGAGGCCAAGGCAGGTGAAGCACCAACTGAGAACCCTGCTCCACCCACC

1081 CAGCAGAGCAGTGCTGAGTAACACCAGGCTCCTCAGGCACCTTCACCATCGGCAGGTGAC

1141 CTAAAGAATTAATGACCATTCAGAAATAAAGCAAAAAGCAGGCCACAACCTTAACCAACA

1201 CCAAAGAAACATCCAAGCAATAAAGTGGAAGACTAACCAAGATTTGGACATTGGAATGTT

1261 TACTGTTATTCTTTAAGAAACAACTACAAAAAGAAAATGTCAACAAATTTTTCAGCAAGC

1321 TGAGAACCTGGGAATTC

die für das zelluläre DNA-bindende Protein RS1, oder ein RS1-ähnliches Polypeptid mit der biologischen Aktivität von RS1 kodiert.

2. DNA-Sequenz, dadurch gekennzeichnet, daß sie aus dem Genom von primären menschlichen Keratinozyten stammt und für das zelluläre Bindungs-Protein RS1 oder ein RS1-ähnliches Polypeptid mit der biologischen Aktivität von RS1 kodiert.

3. DNA-Sequenz, dadurch gekennzeichnet, daß sie mit einer DNA-Sequenz nach den Ansprüchen 1 und 2 hybridisiert, daß sie natürlichen, halb-synthetischen oder synthetischen Ursprungs ist, daß sie in Beziehung zu einer DNA-Sequenz nach den Ansprüchen 1 oder 2 steht durch Mutation wie Nukleotid-Substitution, Nukleotid-Deletion, Nukleotid-Insertion oder Inversion von Nukleotid-Abschnitten, und daß sie für ein RS1-ähnliches Polypeptid mit der biologischen Aktivität von RS1 kodiert.

4. Rekombinantes DNA-Molekül für die Klonierung, dadurch gekennzeichnet, daß die DNA-Sequenz nach einem der Ansprüche 1 bis 3 ausgewählt ist.

5. Rekombinantes DNA-Molekül nach Anspruch 4, dadurch gekennzeichnet, daß die DNA-Sequenz mit einer Expressions-Kontroll-Sequenz operativ verbunden ist.

6. Rekombinantes DNA-Molekül nach Anspruch 5, dadurch gekennzeichnet, daß die Expressions-Kontroll-Sequenz ausgewählt ist aus einem E.coli-Promotersystem, dem E.coli-Lac-System, dem E.coli-ß-Lactamase-System, dem E.coli-trp-System, dem E.coli-Lipoproteinpromoter, einer Hefe-Expressions-Kontroll-Sequenz oder einer anderen eukaryotischen Expressions-Kontroll-Sequenz.

7. Wirtsorganismus, dadurch gekennzeichnet, daß er eine DNA-Sequenz, die für das DNA-bindende Protein RS1 kodiert, enthält.

8. Wirtsorganismus nach Anspruch 7, dadurch gekennzeichnet, daß er mit wenigstens einem der rekombinanten DNA-Moleküle nach einem der Ansprüche 4 bis 6 transformiert ist.

9. Wirtsorganismus nach Anspruch 8, dadurch gekennzeichnet, daß er ausgewählt ist aus der Gruppe E.coli, einem anderen Bakterium, Hefe, einem anderen Pilz, einer tierischen oder menschlichen Zelle.

10. Wirtsorganismus nach Anspruch 9, dadurch gekennzeichnet, daß es sich um Lambda gt 11 handelt.

11. DNA-bindendes Protein RS1 mit der folgenden Aminosäuresequenz:

GluPheArgSerAlaGlnHisGluAlaArgAlaAlaSerAlaAlaArgProHisLeuGly R1

ArgArgArgLeuArgArgGluIleArgProGlyLeuProGluSerGluProArgProPro R1

ArgProProAlaAlaLeuThrAlaAspGlnProArgProSerLeuSerGluSerArgGly R1

GlyGlyGlyMetSerGluAlaGlyGluAlaThrThrThrThrThrThrThrLeuProGln R1

AlaProThrGluAlaAlaAlaAlaAlaProGlnAspProAlaProLysSerProValGly R1

SerGlyAlaProGlnAlaAlaAlaProAlaProAlaAlaHisValAlaGlyAsnProGly R1

GlyAspAlaAlaProAlaAlaThrGlyThrAlaAlaAlaAlaSerLeuGlyAlaAlaAla R1

GlySerGluAspAlaGluLysLysValLeuAlaThrLysValLeuGlyThrValLysTrp R1

PheAsnValArgAsnGlyTyrGlyPheIleAsnArgAsnAspThrLysGluAspValPhe R1

ValHisGlnThrAlaIleLysLysAsnAsnProArgLysTyrLeuArgSerValGlyAsp R1

GlyGluThrValGluPheAspValValGluGlyGluLysGlyAlaGluAlaAlaAsnVal R1

ThrGlyProAspGlyValProValGluGlySerArgTyrAlaAlaAspArgArgArgTyr R1

ArgArgGlyTyrTyrGlyArgArgArgGlyProProArgAsnAlaGlyGluIleGlyGlu R1

MetLysAspGlyValProGluGlyAlaGlnLeuGlnGlyProValHisArgAsnProThr R1

TyrArgProArgTyrArgSerArgGlyProProArgProArgProAlaProSerSerTrp R1

ArgGly***Arg***ArgLysSerAlaSerHisGlnTrpSerLysProAlaValCysSer R1

ProTrpIleProAlaSerLeuGlnLeuProAlaSerProArgProProAsnAlaProSer R1

GlnAspGlyLysGluAlaLysAlaGlyGluAlaProThrGluAsnProAlaProProThr R1

GlnGlnSerSerAlaGlu***HisGlnAlaProGlnAlaProSerProSerAlaGlyAsp R1

LeuLysAsn******ProPheArgAsnLysAlaLysSerArgProGlnPro***ProThr R1

ProLysLysHisProSerAsnLysValGluAsp***ProArgPheGlyHisTrpAsnVal R1

TyrCysTyrSerLeuArgAsnAsnTyrLysLysLysMetSerThrAsnPheSerAlaSer R1

***GluProGlyAsn R1

**12.** DNA-bindendes Protein mit der Eigenschaft, spezifisch an die 28 bp E2-Subregion der Upstream-Regulatory-Region des menschlichen Papillomavirus HPV 18, die die Basensequenz ACCGAAAACGGTCGGGACCGAAAACGGT besitzt, zu binden.

**13.** DNA-bindendes Protein mit der biologischen Aktivität von RS1, dadurch gekennzeichnet, daß es von einer DNA-Sequenz nach einem der Ansprüche 1 bis 3 kodiert wird.

**14.** DNA-bindendes Protein, dadurch gekennzeichnet, daß es mit Hilfe der rekombinanten DNA-Technologie unter Verwendung eines Wirtsorganismus der Ansprüche 7 bis 10 hergestellt wird.

**15.** Verfahren zur Herstellung von RS1 oer RS1-ähnlichen Polypeptiden mit der biologischen Aktivität von RS1, dadurch gekennzeichnet, daß ein Wirtsorganismus mit einem rekombinanten DNA-Molekül nach den Ansprüchen 5 oder 6 transfrormieret wird, der transformierte Organismus kultiviert wird und das RS1 oder RS1-ähnliche Polypeptid nach Expression isoliert wird.

**16.** Polypeptid mit der biologischen Aktivität von RS1, dadurch gekennzeichnet, daß es nach einem Verfahren nach Anspruch 15 erhältlich ist.

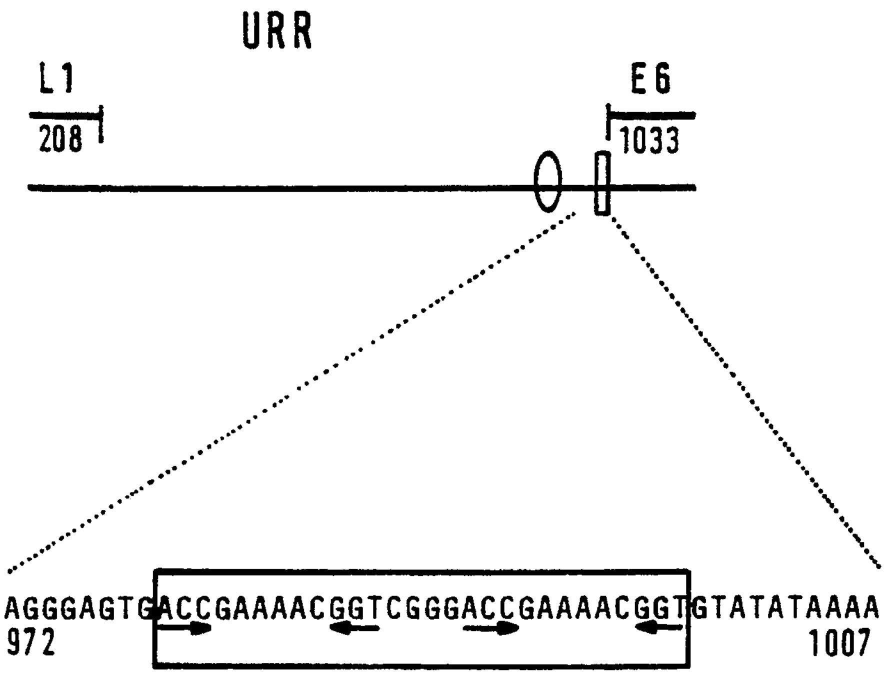
URR
L1
208
E6
1033
AGGGAGTGACCGAAAACGGTCGGGACCGAAAACGGTGTATATAAAA
972
1007

Fig. 1

OLIGONUCLEOTIDES

**Fig. 2**

```
A1:      AGGGAGTCACCGAAAACGGTCGGGACCGAAAACGGTGTAT
         TCCCTCACTGGCTTTTGCCAGCCCTGGCTTTTGCCACATA

A2:  AGCTTGGGAGTGACCGAAAACGGTCGGGACCGAAAACGGTGTATCGGGATCCCG
         ACCCTCACTGGCTTTTGCCAGCCCTGGCTTTTGCCACATAGCCCTAGGGC

A3:         AGCTTACCGAAAACGGTCGGGACCGAAAACGGTACCGAAAACGGTCGGGACCGAAAACGGT
                ATGGCTTTTGCCAGCCCTGGCTTTTGCCATGGCTTTTGCCAGCCCTGGCTTTTGCCAG

B:   ACTTAAGCTAATTGCATACTTGGCTTGTACAACT
     TGAATTCGATTAACGTATGAACCGAACATGTTGA

C:   TGTAATAAAACTGCTTTTAGGCACATATTTTAGTTTGTTTTTA
     ACATTATTTTGACGAAAATCCGTGTATAAAATCAAACAAAAAT

D:   TACAACAATTGCTTGCATAACTATATCCACTCCCTATGT
     ATGTTGTTAACGAACGTATTGATATAGGTGAGGGATACA

E:   AGCTTTTGTATGGTATGTATGGTTGTTGTTGTATGTTGTATGTTACTATAATTGTTGGTATGTGG
         AAACATACCATACATACCAACAACAACATACAACATACAATGATATTAACAACCATACACCG

F:   AGCTTTTGCTTGCATAATTGCTTGCATAATTGCTTGCATAATTGCTTGCATAAG
         AAACGAACGTATTAACGAACGTATTAACGAACGTATTAACGAACGTATTCCTAG

G:   AGCTTGCATTTTTGGCTTGTAGCATTTTTGGCTTGTAGCATTTTTGGCTTGTAGCATTTTTGGCTTGTAG
         ACGTAAAAACCGAACATCGTAAAAACCGAACATCGTAAAAACCGATCATCGTAAAAACCGAACATCCTAG
```

# Fig. 3A

```
  1  GAATTCCGGAGCGCGCAGCACGAAGCTCGAGCCGCCTCCGCCGCGCGACCCCACCTCGGC
     GluPheArgSerAlaGlnHisGluAlaArgAlaAlaSerAlaAlaArgProHisLeuGly    R1

 61  CGCCGCCGCCTGCGCCGCGAGATCCGCCCCGGCCTCCCCGAGAGCGAGCCCCGGCCGCCG
     ArgArgArgLeuArgArgGluIleArgProGlyLeuProGluSerGluProArgProPro    R1

121  CGACCACCAGCCGCGCTAACCGCCGACCAACCGCGACCGAGCCTGAGCGAGAGCAGAGGA
     ArgProProAlaAlaLeuThrAlaAspGlnProArgProSerLeuSerGluSerArgGly    R1

181  GGAGGAGGCATGAGTGAGGCGGGCGAGGCCACCACCACCACCACCACCACCCTCCCGCAG
     GlyGlyGlyMetSerGluAlaGlyGluAlaThrThrThrThrThrThrThrLeuProGln    R1

241  GCTCCGACGGAGGCGGCCGCCGCGGCTCCCCAGGACCCCGCGCCCAAGAGCCCGGTGGGC
     AlaProThrGluAlaAlaAlaAlaAlaProGlnAspProAlaProLysSerProValGly    R1

301  GGGGACGCGGCCCCTGCAGCCACGGGCACCGCGGCCGCCGCCTCTTTAGGCGCCGCCGCC
     SerGlyAlaProGlnAlaAlaAlaProAlaProAlaAlaHisValAlaGlyAsnProGly    R1

361  GGGGACGCGGCCCCTGCAGCCACGGGCACCGCGGCCGCCGCCTCTTTAGGCGCCGCCGCC
     GlyAspAlaAlaProAlaAlaThrGlyThrAlaAlaAlaAlaSerLeuGlyAlaAlaAla    R1

421  GGCAGCGAAGACGCGGAGAAAAAGTTCTCGCCACCAAAGTCCTTGGCACTGTCAAATGG
     GlySerGluAspAlaGluLysLysValLeuAlaThrLysValLeuGlyThrValLysTrp    R1

481  TTCAACGTCAGAAATGGATATGGATTTATAAATCGAAATGACACCAAAGAAGATGTATTT
     PheAsnValArgAsnGlyTyrGlyPheileAsnArgAsnAspThrLysGluAspValPhe    R1

541  GTACATCAGACTGCCATCAAGAAGAATAACCCACGGAAATATCTGCGCAGTGTAGGAGAT
     ValHisGlnThrAlaIleLysLysAsnAsnProArgLysTyrLeuArgSerValGlyAsp    R1

601  GGAGAAACTGTAGAGTTTGATGTGGTTGAAGGAGAGAAGGGTGCAGAAGCTGCCAATGTG
     GlyGluThrValGluPheAspValValGluGlyGluLysGlyAlaGluAlaAlaAsnVal    R1
```

```
661   ACTGGCCCGGATGGAGTTCCTGTGGAAGGGAGTCGTTACGCTGCAGATCGGCGCCGTTAC
      ThrGlyProAspGlyValProValGluGlySerArgTyrAlaAlaAspArgArgArgTyr   R1

721   AGACGTGGCTACTATGGAAGGCGCCGTGGCCCTCCCCGGAATGCTGGTGAGATTGGAGAG
      ArgArgGlyTyrTyrGlyArgArgArgGlyProProArgAsnAlaGlyGluIleGlyGlu   R1

781   ATGAAGGATGGAGTCCCAGAGGGAGCACAACTTCAGGGACCGGTTCATCGAAATCCAACT
      MetLysAspGlyValProGluGlyAlaGlnLeuGlnGlyProValHisArgAsnProThr   R1

841   TACCGCCCAAGGTACCGTAGCAGGGGACCTCCTCGCCCACGACCTGCCCCCAGCAGTTGG
      TyrArgProArgTyrArgSerArgGlyProProArgProArgProAlaProSerSerTrp   R1

901   AGAGGCTGAAGATAAAGAAAATCAGCAAGCCACCAGTGGTCCAAACCAGCCGTCTGTTCG
      ArgGly***Arg***ArgLysSerAlaSerHisGlnTrpSerLysProAlaValCysSer   R1

961   CCGTGGATACCAGCGTCCCTACAATTACCGGCGTCGCCGCGTCCTCCTAACGCTCCTTCA
      ProTrpIleProAlaSerLeuGlnLeuProAlaSerProArgProProAsnAlaProSer   R1
1021  CAAGATGGCAAAGAGGCCAAGGCAGGTGAAGCACCAACTGAGAACCCTGCTCCACCCACC
      GlnAspGlyLysGluAlaLysAlaGlyGluAlaProThrGluAsnProAlaProProThr   R1

1081  CAGCAGAGCAGTGCTGAGTAACACCAGGCTCCTCAGGCACCTTCACCATCGGCAGGTGAC
      GlnGlnSerSerAlaGlu***HisGlnAlaProGlnAlaProSerProSerAlaGlyAsp   R1

1141  CTAAAGAATTAATGACCATTCAGAAATAAAGCAAAAAGCAGGCCACAACCTTAACCAACA
      LeuLysAsn******ProPheArgAsnLysAlaLysSerArgProGlnPro***ProThr   R1

1201  CCAAAGAAACATCCAAGCAATAAAGTGGAAGACTAACCAAGATTTGGACATTGGAATGTT
      ProLysLysHisProSerAsnLysValGluAsp***ProArgPheGlyHisTrpAsnVal   R1

1261  TACTGTTATTCTTTAAGAAACAACTACAAAAAGAAAATGTCAACAAATTTTTCAGCAAGC
      TyrCysTyrSerLeuArgAsnAsnTyrLysLysLysMetSerThrAsnPheSerAlaSer   R1

1321  TGAGAACCTGGGAATTC
      ***GluProGlyAsn                                                R1
```

Fig. 3B

Europäisches Patentamt

# EUROPÄISCHER RECHERCHENBERICHT

Nummer der Anmeldung

EP 91 10 4659

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | VIROLOGY<br>vol. 181, no. 1, 28 Februar 1991,<br>Seiten 132 - 138; AUBORN,KJ & STEINBERG,BM:<br>"Akey DNA-protein interaction determines the function of the 5'URR enhancer in Human Papillomavirus Type 11"<br>* das ganze Dokument *<br>--- | 3, 13 | C07K13/00<br>C12N15/12<br>A61K37/02 |
| P,A | JOURNAL OF VIROLOGY<br>vol. 64, no. 10, Oktober 1990,<br>Seiten 4743 - 4754; BOSCH, F.X. et al.:<br>"Suppression in vivo of Human Papillomavirus Type+8 E6-E7 gene expression in non tumorigenic HeLa x Fibroblast hybrid cells"<br>--- | 7 | |
| A | JOURNAL OF CELLULAR BIOCHEMISTRY<br>vol. SUPPL, no. 13, 19 März 1989,<br>Seite 203 BARTSCH, D et al.:<br>"Constitutive expression of Human Papillomavirus Type 18 (HPV18) early genes driven by Human Cytomegalovirus (HCMV) early promoter results i n tumorigenic conversion of nontumorigenic HeLa x Fibroblasts human"<br>* Zusammenfassung * 1202 *<br>--- | 7 | **RECHERCHIERTE SACHGEBIETE (Int. Cl.5)**<br>C07K |
| A | JOURNAL OF VIROLOGY<br>vol. 61, no. 5, 08 Mai 1987,<br>Seiten 1655 - 1660; MALLON, R.G. et al.:<br>"DNA-Binding activity of Papillomavirus proteins "<br>* das ganze Dokument *<br>--- | 12 | |
| X | GENE.<br>vol. 73, 1988, AMSTERDAM NL<br>Seiten 499 - 507; SAKURA, H. et al.:<br>"Two human genes isolated by a novel method encode Dna-binding proteins containing a common region of homology"<br>* das ganze Dokument *<br>----- | 3 | |

Der vorliegende Recherchenbericht wurde für alle Patentansprüche erstellt

| Recherchenort | Abschlußdatum der Recherche | Prüfer |
|---|---|---|
| DEN HAAG | 04 JULI 1991 | CHAMBONNET F.J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

INFORMATION FOR SEQ ID NO:1:

(i) SEQUENCE CHARACTERISTICS:
(A) LENGTH: 1337 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

```
GAATTCCGGA GCGCGCAGCA CGAAGCTCGA GCCGCCTCCG CCGCGCGACC CCACCTCGGC
CGCCGCCGCC TGCGCCCGGA GATCCGCCCC GGCCTCCCCC AGAGCGAGCC CCGGCCGCCG
CGACCACCAG CCGCGCTAAC CGCCGACCAA CCGCGACCGA GCCTGAGCGA GAGCAGAGGA
GGAGGAGGCA TGAGTGAGGC GGGCGAGGCC ACCACCACCA CCACCACCAC CCTCCCGCAG
GCTCCGACGG AGGCGGCCGC CGCGGCTCCC CAGGACCCCG CGCCCAAGAG CCCGGTGGGC
AGCGGTGCGC CCCAGGCCGC GGCCCCGGCG CCCGCCCCCC ACGTCGCAGG AAACCCCGGT
GGGGACGCGG CCCCTGCACC CAGGGGCACC GCGGCCGCCG CCTCTTTAGG CGCCGCCGCC
GGCAGCGAAG ACGCGGAGAA AAAAGTTCTC GCCACCAAAG TCCTTGGCAC TGTCAAATGG
TTCAACGTCA GAAATGGATA TGGATTTATA AATCGAAATG ACACCAAAGA AGATGTATTT
CTACATCAGA CTGCCATCAA GAAGAATAAC CCACGGAAAT ATCTGCGCAG TGTAGGAGAT
GGAGAAACTG TAGAGTTTGA TGTGGTTGAA GGAGAGAAGG GTGCAGAAGC TGCCAATGTG
ACTGGCCCGG ATGGAGTTCC TGTGGAAGGG AGTCGTTACG CTGCAGATCG GCGCCGTTAC
AGACGTGGCT ACTATGGAAG GCGCCGTGGC CCTCCCCGGA ATGCTGGTGA GATTGGAGAG
ATGAAGGATG GAGTCCCAGA GGGAGCACAA CTTCAGGGAC CGGTTCATCG AAATCCAACT
TACCGCCCAA GGTACCGTAG CAGGGGACCT CCTCGCCCAC GACCTGCCCC CAGCAGTTGG
AGAGGCTGAA GATAAAGAAA ATCAGCAAGC CACCAGTGGT CCAAACCAGC CGTCTGTTCG
CCGTGGATAC CAGCGTCCCT ACAATTACCG GCGTCGCCGC GTCCTCCTAA CGCTCCTTCA
CAAGATGGCA AAGAGGCCAA GGCAGGTGAA GCACCAACTG AGAACCCTGC TCCACCCACC
CAGCAGAGCA GTGCTGAGTA ACACCAGGCT CCTCAGGCAC CTTCACCATC GGCAGGTGAC
CTAAAGAATT AATGACCATT CAGAAATAAA GCAAAAAGCA GGCCACAACC TTAACCAACA
CCAAAGAAAC ATCCAAGCAA TAAAGTGGAA GACTAACCAA GATTTGGACA TTGGAATGTT
TACTGTTATT CTTTAAGAAA CAACTACAAA AAGAAAATGT CAACAAATTT TTCAGCAAGC
TGAGAACCTG GGAATTC
```

INFORMATION FOR SEQ ID NO:2:

(i). SEQUENCE CHARACTERISTICS:
(A) LENGTH: 390 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

GAGAAGGAAA GAAAAAAAAA GGAGAGAGAG AGGAGGGGAG AGAAAATAAA GAGGAGGGGG

TGAAGGAATT TTTTTGAATG AAAAAAAAAA GAAAAGGAAG AAAAAAAAAA AAAAAGAGGA

AAAAAAAATG TAAAAAAAAG AAAAAAGGAA AGAAATAGTA TAAAGAGTGA AGAATGAAAG

TAAAAAGTAG AGAGGTAGAA AGGGGAAGAA AAAATGAGAA GGAGTAAAAA AGAGAGTAGA

GGTTGAGAGA GGAGAAAGGG GTAGAGGAAG GGAAGATTAG AGTAGAGGAG AGAATAGGAG

AGAAGTAAAC TAAGAAAGAA AGAGGAAAAG GAATGAAATG GAAGGAAGAA AAGAAAGAAA

AGGTAAGAAG GTAATCTAGA ATTTAAAGGA

INFORMATION FOR SEQ ID NO:3:

(i) SEQUENCE CHARACTERISTICS:
(A) LENGTH: 437 amino acids
(B) TYPE: amino acid
(D) TOPOLOGY: linear

(ii) MOLECULE TYPE: peptide

(xi) SEQUENCE DESCRIPTION: SEQ ID NO:3:

```
Glu Phe Arg Ser Ala Gln His Glu Ala Arg Ala Ala Ser Ala Ala Arg
1               5                   10                  15

Pro His Leu Gly Arg Arg Arg Leu Arg Arg Glu Ile Arg Pro Gly Leu
            20                  25                  30

Pro Glu Ser Glu Pro Arg Pro Pro Arg Pro Pro Ala Ala Leu Thr Ala
        35                  40                  45

Asp Gln Pro Arg Pro Ser Leu Ser Glu Ser Arg Gly Gly Gly Gly Met
    50                  55                  60

Ser Glu Ala Gly Glu Ala Thr Thr Thr Thr Thr Thr Thr Leu Pro Gln
65                  70                  75                  80

Ala Pro Thr Glu Ala Ala Ala Ala Ala Pro Gln Asp Pro Ala Pro Lys
                85                  90                  95
```

```
Ser Pro Val Gly Ser Gly Ala Pro Gln Ala Ala Ala Pro Ala Pro Ala
            100                 105                 110

Ala His Val Ala Gly Asn Pro Gly Gly Asp Ala Ala Pro Ala Ala Thr
        115                 120                 125

Gly Thr Ala Ala Ala Ala Ser Leu Gly Ala Ala Ala Gly Ser Glu Asp
    130                 135                 140

Ala Glu Lys Lys Val Leu Ala Thr Lys Xaa Leu Gly Thr Xaa Lys Trp
145                 150                 155                 160

Phe Asn Val Arg Asn Gly Tyr Gly Phe Ile Asn Arg Asn Asp Thr Lys
                165                 170                 175

Lys Tyr Leu Arg Ser Xaa Gly Asp Gly Glu Thr Xaa Glu Phe Asp Val
        195                 200                 205

Val Glu Gly Glu Lys Gly Ala Glu Ala Ala Asn Xaa Thr Gly Pro Asp
    210                 215                 220

Gly Xaa Pro Xaa Glu Gly Ser Arg Tyr Ala Ala Asp Arg Arg Arg Tyr
225                 230                 235                 240

Arg Arg Gly Tyr Tyr Gly Arg Arg Arg Gly Pro Pro Arg Asn Ala Gly
                245                 250                 255

Glu Ile Gly Glu Met Lys Asp Gly Xaa Pro Glu Gly Ala Gln Leu Gln
            260                 265                 270

Gly Pro Xaa His Arg Asn Pro Thr Tyr Arg Pro Arg Tyr Arg Ser Arg
        275                 280                 285

Gly Pro Pro Arg Pro Arg Pro Ala Pro Ser Ser Trp Arg Gly Arg Arg
    290                 295                 300

Lys Ser Ala Ser His Gln Trp Ser Lys Pro Ala Xaa Cys Ser Pro Trp
305                 310                 315                 320

Ile Pro Ala Ser Leu Gln Leu Pro Ala Ser Pro Arg Pro Pro Asn Ala
                325                 330                 335

Pro Ser Gln Asp Gly Lys Glu Ala Lys Ala Gly Glu Ala Pro Thr Glu
            340                 345                 350

Asn Pro Ala Pro Pro Thr Gln Gln Ser Ser Ala Glu His Gln Ala Pro
        355                 360                 365

Gln Ala Pro Ser Pro Ser Ala Gly Asp Leu Lys Asn Pro Phe Arg Asn
    370                 375                 380

Lys Ala Lys Ser Arg Pro Gln Pro Pro Thr Pro Lys Lys His Pro Ser
385                 390                 395                 400

Asn Lys Xaa Glu Asp Pro Arg Phe Gly His Trp Asn Xaa Tyr Cys Tyr
                405                 410                 415

Ser Leu Arg Asn Asn Tyr Lys Lys Lys Met Ser Thr Asn Phe Ser Ala
            420                 425                 430

Ser Glu Pro Gly Asn
        435
```

INFORMATION FOR SEQ ID NO:4:

(i) SEQUENCE CHARACTERISTICS:
(A) LENGTH: 80 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

AGGGAGTCAC CGAAAACGGT CGGGACCGAA AACGGTGTAT TCCCTCACTG GCTTTTGCCA

GCCCTGGCTT TTGCCACATA

INFORMATION FOR SEQ ID NO:5:

(i) SEQUENCE CHARACTERISTICS:
(A) LENGTH: 104 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

AGCTTGGGAG TGACCGAAAA CGGTCGGGAC CGAAAACGGT GTATCGGGAT CCCGACCCTC

ACTGGCTTTT GCCAGCCCTG GCTTTTGCCA CATAGCCCTA GGGC

INFORMATION FOR SEQ ID NO:6:

(i) SEQUENCE CHARACTERISTICS:
(A) LENGTH: 119 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

AGCTTACCGA AAACGGTCGG GACCGAAAAC GGTACCGAAA ACGGTCGGGA CCGAAAACGG

TATGGCTTTT GCCAGCCCTG GCTTTTGCCA TGGCTTTTGC CAGCCCTGGC TTTTGCCAG

INFORMATION FOR SEQ ID NO:7:

(i) SEQUENCE CHARACTERISTICS:
(A) LENGTH: 68 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

ACTTAAGCTA ATTGCATACT TGGCTTGTAC AACTTGAATT CGATTAACGT ATGAACCGAA

CATGTTGA

INFORMATION FOR SEQ ID NO:8:

(i) SEQUENCE CHARACTERISTICS:
(A) LENGTH: 86 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:8:

TGTAATAAAA CTGCTTTTAG GCACATATTT TAGTTTGTTT TTAACATTAT TTTGACGAAA

ATCCGTGTAT AAAATCAAAC AAAAAT

INFORMATION FOR SEQ ID NO:9:

(i) SEQUENCE CHARACTERISTICS:
(A) LENGTH: 78 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:9:

TACAACAATT GCTTGCATAA CTATATCCAC TCCCTATGTA TGTTGTTAAC GAACGTATTG

ATATAGGTGA GGGATACA

INFORMATION FOR SEQ ID NO:10:

(i) SEQUENCE CHARACTERISTICS:
(A) LENGTH: 127 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

AGCTTTTGTA TGGTATGTAT GGTTGTTGTT GTATGTTGTA TGTTACTATA ATTGTTGGTA

TGTGGAAACA TACCATACAT ACCAACAACA ACATACAACA TACAATGATA TTAACAACCA

TACACCG

INFORMATION FOR SEQ ID NO:11:

(i) SEQUENCE CHARACTERISTICS:
(A) LENGTH: 108 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

AGCTTTTGCT TGCATAATTG CTTGCATAAT TGCTTGCATA ATTGCTTGCA TAAGAAACGA

ACGTATTAAC GAACGTATTA ACGAACGTAT TAACGAACGT ATTCCTAG

INFORMATION FOR SEQ ID NO:12:

(i) SEQUENCE CHARACTERISTICS:
(A) LENGTH: 140 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

AGCTTGCATT TTTGGCTTGT AGCATTTTTG GCTTGTAGCA TTTTTGGCTT GTAGCATTTT

TGGCTTGTAG ACGTAAAAAC CGAACATCGT AAAAACCGAA CATCGTAAAA ACCGATCATC

GTAAAAACCG AACATCCTAG